# EUROPEAN PATENT APPLICATION

(11) **EP 0 921 192 A1**
(43) Date of publication of application: **09.06.1999**
(21) Application number: 97203781.6
(22) Date of filing: 03.12.1997
(51) Int. Cl.: C12N 15/12, C07K 14/47, A61K 38/17, C12Q 1/68, C12N 1/19, C12N 1/21

(54) **Molecules interacting with apoptin**

(71) Applicant: Leadd B.V., 2333 AL Leiden (NL)
(72) Inventor: Noteborn, Mathieu Hubertus Maria, 2352 EH Leiderdorp (NL); Danen - van Oorschot, Astrid A. A. M., 2651 VG Berkel en Rodenrijs (NL)
(74) Representative: Smulders, Theodorus A.H.J., Ir.

(57) **Abstract**

The invention relates to activation of apoptosis by means of interference of Hou-like and/or IFP35-like compounds.

Also the invention relates to anti-tumor therapies with compounds, which negatively interfere with Hou-like and/or IFP35-like compounds leading to induction of apoptosis, resulting in the elimination of tumor cells.

Also the invention relates to therapies for diseases related to aberrant apoptosis induction, such as auto-immune disease.

Also the invention describes the diagnosis of cells, which are susceptible to apoptin- or apoptin-like induced apoptosis.

## Description

The present invention relates to the field of apoptosis, as well as to the field of cancer diagnosis and treatment, and treatment and diagnosis of (auto-) immune diseases and other diseases related to regulation of apoptosis. The present invention specifically relates to molecules found to be involved in apoptotic pathways and their uses in inducing apoptosis in aberrant cells. The presently invented molecules have been identified using apoptin. Apoptin is a protein originally found in chicken anemia virus (CAV; Noteborn et al., 1991) and was originally called VP3. The apoptotic activity of this protein was discovered by the group of the present inventors (Noteborn et al., 1994).

Apoptosis is an active and programmed physiological process for eliminating superfluous, altered or malignant cells (Earnshaw, 1995, Duke et al., 1996). Apoptosis is characterized by shrinkage of cells, segmentation of the nucleus, condensation and cleavage of DNA into domain-sized fragments, in most cells followed by internucleosomal degradation. The apoptotic cells fragment into membrane-enclosed apoptotic bodies. Finally, neighbouring cells and/or macrophages will rapidly phagocytose these dying cells (Wyllie et al., 1980, White, 1996). Cells grown under tissue-culture conditions and cells from tissue material can be analysed for being apoptotic with agents staining DNA, as e.g. DAPI, which stains normal DNA strongly and regularly, whereas apoptotic DNA is stained weakly and/or irregularly (Noteborn et al., 1994, Telford et al., 1992).

The apoptotic process can be initiated by a variety of regulatory stimuli (Wyllie, 1995, White 1996, Levine, 1997). Changes in the cell survival rate play an important role in human pathogenesis, e.g. in cancer development and auto-immune diseases, which is caused by enhanced proliferation but also by decreased cell death (Kerr et al., 1994, Paulovich, 1997). A variety of chemotherapeutic compounds and radiation have been demonstrated to induce apoptosis in tumor cells, in many instances via wild-type p53 protein (Thompson, 1995, Bellamy et al., 1995, Steller, 1995, McDonell et al., 1995).

Many tumors, however, acquire a mutation in p53 during their development, often correlating with poor response to cancer therapy. Transforming genes of tumorigenic DNA viruses can inactivate p53 by directly binding to it (Teodoro, 1997). An example of such an agent is the large T antigen of the tumor DNA virus SV40. For several (leukemic) tumors, a high expression level of the proto-oncogene Bcl-2 or Bcr-abl is associated with a strong resistance to various apoptosis-inducing chemotherapeutic agents (Hockenberry 1994, Sachs and Lotem, 1997).

For such cancers (representing more than half of the tumors) alternative anti-tumor therapies are under development based on induction of apoptosis independent of p53 (Thompson 1995, Paulovich et al., 1997). One has to search for the factors involved in induction of apoptosis, which do not need p53 and/or can not be blocked by Bcl-2/Bcr-abl-like anti-apoptotic activities. These factors might be part of a distinct apoptosis pathway or might be (far) downstream to the apoptosis inhibiting compounds.

Apoptin is a small protein derived from chicken anemia virus (CAV; Noteborn and De Boer, 1995, Noteborn et al., 1991, Noteborn et al., 1994), which can induce apoptosis in human malignant and transformed cell lines, but not in untransformed human cell cultures. In vitro, apoptin fails to induce programmed cell death in normal lymphoid, dermal, epidermal, endothelial and smooth-muscle cells. However, when normal cells are transformed they become susceptible to apoptosis by apoptin. (Danen-van Ooschot, 1997 and Noteborn, 1996). Long-term expression of apoptin in normal human fibroblasts revealed that apoptin has no toxic or transforming activity in these cells.

In normal cells, apoptin was found predominantly in the cytoplasm, whereas in transformed or malignant cells i.e. characterized by hyperplasia, metaplasia or dysplasia, it was located in the nucleus, suggesting that the localization of apoptin is related to its activity (Danen-van Oorschot et al. 1997).

Apoptin-induced apoptosis occurs in the absence of functional p53 (Zhuang et al., 1995a), and cannot be blocked by Bcl-2, Bcr-abl (Zhuang et al., 1995), the Bcl-2-associating protein BAG-1 and not by the caspase-inhibitor cowpox protein CrmA (Danen-Van Oorschot, 1997a, Noteborn, 1996).

Therefore, apoptin is useful for the destruction of tumor cells, or other hyperplasia, metaplasia or dysplasia which have become resistant to (chemo)therapeutic induction of apoptosis, due to the lack of functional p53 and (over)expression of Bcl-2 and other apoptosis-inhibiting agents (Noteborn et al., 1997).

The fact that apoptin does not induce apoptosis in normal human cells, at least not in vitro, suggests that a toxic effect of apoptin treatment in vivo will be very low. Noteborn et al. (1997) have provided evidence that adenovirus expressed apoptin does not have an acute toxic effect in vivo. In addition, in nude mice it was shown that apoptin has a strong anti-tumor activity.

It appears, that even pre-malignant, minimally transformed cells, are sensitive to the death-inducing effect of apoptin. In addition, Noteborn and Zhang (1997) have shown that apoptin-induced apoptosis can be used as diagnosis of cancer-prone cells and treatment of cancer-prone cells.

Knowing that apoptin is quite safe in normal cells, but that as soon as a cell becomes transformed and/or immortalized (the terms may be used interchangeable herein) the present inventors designed some uses based on the identification of compounds involved in the apoptin-induced apoptotic cascade. These compounds are factors of an apoptosis pathway, which is specific for transformed cells. Therefore, these proteins are very important compounds in new treatments and diagnosis for diseases related with aberrancies in the apoptotic process, such as cancer and auto-immune diseases.

Proteins found associating with apoptin include members of the family of Nmi/Hou-like and IFP-like proteins.

Thus the invention provides a recombinant and/or isolated nucleic acid molecule encoding at least a functional part of a member of the family of Nmi-like proteins or at least a functional part of a member of the family of Hou-like proteins or at least a functional part of a member of the family of IFP35-like proteins for use in the induction of apoptosis in a population of cells related to a pathological condition.

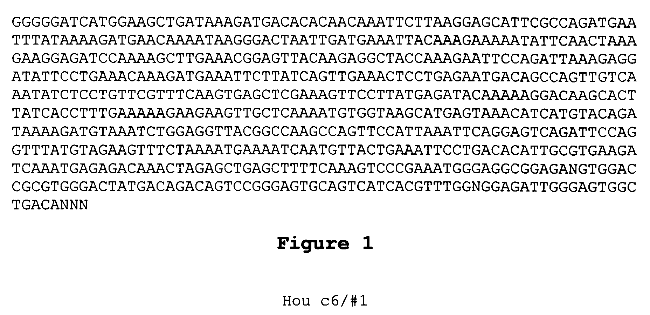

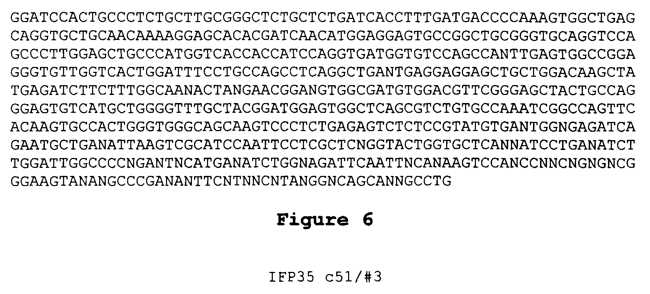

As explained herein the expression of Hou is connected to oncogenes and has been found to be high in certain transformed cells. These are typically the cells that can be induced to go into apoptosis by apoptotic agents such as apoptin. Typically providing a cell with Hou-like activity will therefor increase the chance of inducing apoptosis in such a cell. IFP35-like proteins are involved in transporting apoptotic substances to the nucleus of cells. Under influence of for instance interferons these proteins localize in the nucleus. Therefor IFP-like activity is used to get apoptin-like activity into the nucleus, which is important for the induction of apoptosis, for instance through Hou-like proteins. The Hou-like activity or Nmi-like activity is defined herein as any molecule capable of exerting the sanme or a similar function as the original Hou-like (Nmi-like) protein. The same definition goes for IFP-activity. Typically such a molecule can be encoded by a nucleic acid molecule which comprises at least a functional and specific part of the sequence of figure 1, 2, 4 or 5 or encoding an amino sequence of figure 6 or a sequence at least 60, preferably 70, preferably 90 % homologous with said functional and specific sequence or comprising a sequence hybridizing to any of the aforegoing sequences under stringent conditions. In order to be able to express the Hou-like activity and/or the IFP-like activity it is preferred to have an expression vector encoding said activity. Expression vectors are nucleic acid molecules which can be brought into cells, or transfect cells themselves and which have the machinery (together with the machinery of the host cell) to express proteins encoded on the expression vector when present in a cell.

It is preferred that cells which are provided, according to the invention, with Hou-like activity and/or IFP-like activity, are also provided with apoptosis inducing activity, preferably apoptin-like activity, which is defined along the same lines as Hou-like activity. In order to get the activity into the cells in which apoptosis has to be induced it is possible and preferred to use a gene delivery vehicle. A gene delivery vehicle is a means to transport a nucleic acid molecule capable of expressing the wanted activity in a host cell into said host cell. Gene delivery vehicles are known in the art. They include for instance recombinant viruses such as adenoviruses and retroviruses, but also non-viral vehicles such as polymers and liposomes have been suggested. Methods of targeting gene delivery vehicles to target cells are also known in the art and need not be elaborated herein. The invention also provides the newly identified molecules themselves, both the nucleic acid molecules (meaning DNA coding and/or non coding strands as well as RNA) and the proteinaceous molecules (peptides, polypeptides, glycoproteins and associations between prtoeins and RNA's and the like). Based on the given sequences other familymembers of the Hou/Nmi and IFP families will be identified having the same or similar function. Typically such molecules will have high homology to the sequences given herein.

For nucleic acid molecules the homology is exppected to be at least 60, preferably 70, more preferably 80%. therewith.

These nucleic acid molecules can of course again be incorporated into expression vectors as mentioned hereinbefore. Preferably these expression vectors also encode apoptotic activity, preferably apoptin or a functional fragment and/or equivalent thereof.

These expression vectors can again be made into gene delivery vehicles.

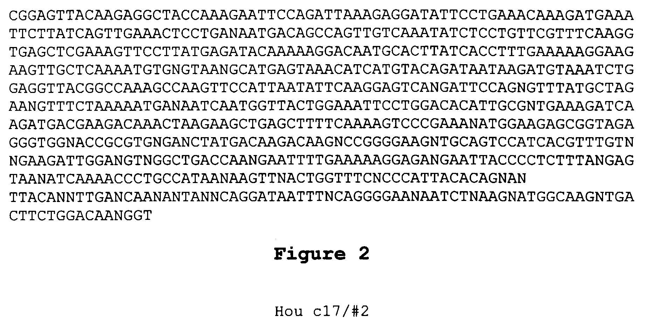

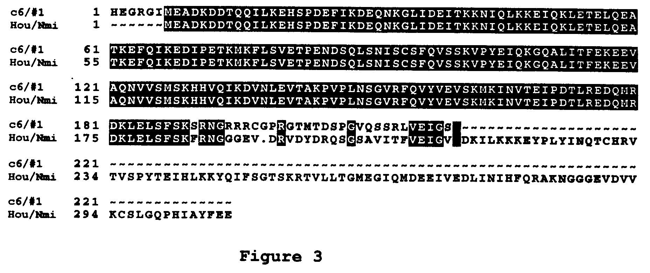

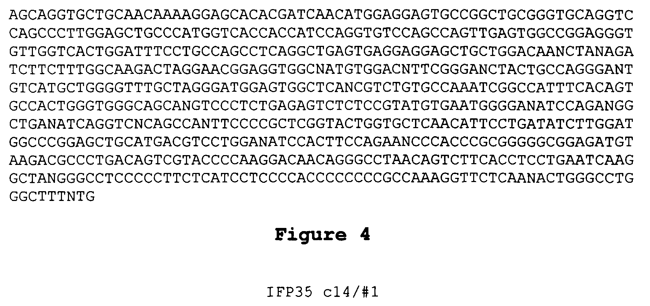

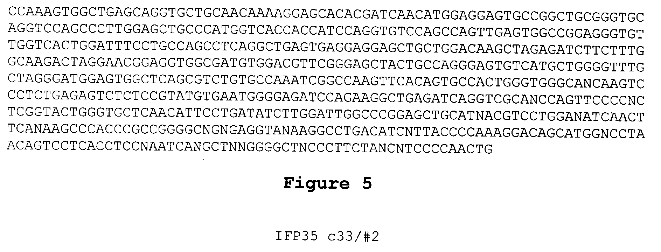

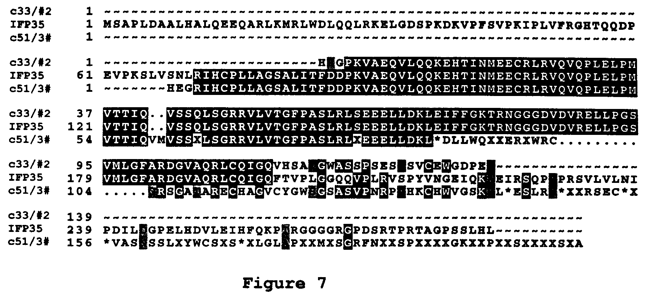

The invention also provides the recombinant or isolated proteinaceous substance comprising at least a functional part of a member of the family of Nmi/Hou-like proteins or at least a functional part of a member of the family of Hou-like proteins for use in the induction of apoptosis in a population of cells related to a pathological condition and an Nmi/Hou-like proteinaceous substance having at least a functional and/or specific part of the sequence of figure 3 or being encoded by a functional and/or specific part of the sequence of figure 1 or figure 2 or being at least 60, preferably 70, preferably 80% homologous to at least a functional and/or specific part of the sequence of figure 3 or being at least 60, preferably 70, preferably 80% homologous to a protein encoded by at least a functional and/or specific part of the sequence of figure 1 or figure 2 and an IFP35-like proteinaceous substance having at least a functional and/or specific part of the sequence of figure 6 or 7 or being encoded by a functional and/or specific part of the sequence of figure 4 or figure 5 or being at least 60, preferably 70, preferably 80% homologous to at least a functional and/or specific part of the sequence of figure 6 or 7 or being at least 60, preferably 70, preferably 80% homologous to a protein encoded by at least a functional and/or specific part of the sequence of figure 4 or figure 5.

A functional part in this document means having the same or similar activity (although the amount of activity may differ) A specific part herein means a part of sufficient size to be specific for the protein or nucleic acid or to be of sufficient size to distinguish the protein from another protein immunologically. The proteins disclosed herein can for instance also be used to identify further components of the apoptotic pathway.

The reason for bringing IFP-like activity and/or Hou-like activity together with apoptotic activity is of course to induce aberrant cells to go into apoptosis. Thus the invention also provides a method for inducing apoptosis in cells comprising providing said cells with Nmi/Hou-like protein activity and/or IFP-35-like activity together with apoptin-like activity.

The invention further provides a method for inducing apoptosis through interference with the function of Nmi/Hou-like proteins (interchangeably referred as Hou-, Nmi- or Nmi/Hou-like proteins).

The invention provides an anti-tumor therapy based on the interference with the function of Hou or Hou-like proteins. The fact that Hou or Hou-like proteins are abundantly present in tumor cells in combination with highly expressed oncogenes, - which are activated by Hou or Hou-like proteins -, make Hou and/or Hou-like proteins very important targets of an anti-tumor agent/therapy.

The invention provides the mediator of apoptin-induced apoptosis, which is tumor-specific.

The invention provides a therapy for cancer, auto-immune diseases or related diseases which is based on Hou-like proteins in combination with apoptin and/or apoptin-like compounds.

The invention further provides a method for inducing apoptosis through interference with the function of IFP35-like proteins.

The invention provides an anti-tumor therapy based on the interference with the function of IFP35 or IFP35-like proteins.

The invention provides IFP35 as a mediator of apoptin-induced apoptosis, which is tumor-specific.

The invention provides a therapy for cancer, auto-immune diseases or related diseases which is based on IFP35 or IFP35-like proteins in combination with apoptin and/or apoptin-like compounds.

The invention provides a therapy based on the combination of apoptin-associating proteins Hou and IFP35.

The invention further provides a method for inducing apoptosis through interference with the function of IFP35 and Hou or IFP35-and Hou-like proteins.

The invention provides an anti-tumor therapy based on the interference with the function of IFP35 or IFP35-like proteins, in combination with Hou or Hou-like proteins.

The invention provides IFP35 or IFP35-like in combination with Hou or Hou-like proteins as mediators of apoptin or apoptin-like induction of apoptosis, which is tumor-specific.

The invention provides a therapy for cancer, auto-immune diseases or related diseases which is based on the combination of IFP35 or IFP35-like and Hou or Hou-like proteins in combination with apoptin and/or apoptin-like compounds.

Furthermore, the invention provides a diagnosis, based on Hou or Hou-like proteins, which can determine whether cells are susceptible for apoptin-induced apoptosis. This means that patients with tumors having up-regulated Hou or Hou-like activity are optimal candidates for an anti-cancer therapy based on apoptin.

Furthermore, the invention provides a diagnosis, based on Hou or Hou-like proteins in combination with IFP35 or IFP35-like proteins, which can determine whether cells are susceptible for apoptin-induced apoptosis. This means that patients with tumors having up-regulated Hou or Hou-like activity in combination with IFP35 or IFP35-like activity are optimal candidates for an anti-cancer therapy based on apoptin.

The invention will be explained in more detail in the following experimental part. This only serves for the purpose of illustration and should not be interpreted as a limitation of the scope of the invention.

### EXPERIMENTAL PART

The inventors have used the yeast-2 hybrid system (Durfee et al., 1993) to identify apoptin-associating cellular compounds, which are essential in the induction of apoptosis. The used system is an in vivo strategy to identify human proteins capable of physically associating with apoptin. It has been used to screen cDNA libraries for clones encoding proteins capable of binding to a protein of interest (Fields and Song, 1989, Yang et al., 1992).

### Construction of pGBT9-VP3

For the construction of the bait plasmid, which enables the identification of apoptin-associating proteins by means of a yeast-two-hybrid system, plasmid pET-16b-VP3 (Noteborn, unpublished results) was treated with NdeI and BamHI. The 0.4 kb NdeI-BamHI DNA fragment was isolated from low-melting-point agarose.

Plasmid pGBT9 (Clontech Laboratories, Inc, Palo Alto, USA) was treated with the restriction enzymes EcoRI and BamHI. The about 5.4-kb DNA fragment was isolated and ligated to an EcoRI-NdeI linker and the 0.4-kb DNA fragment containing the apoptin-encoding sequences starting from its own ATG-initiation codon. The final construct containing a fusion gene of the GAL4-binding domain sequence and apoptin under the regulation of the yeast promoter ADH was called pGBT-VP3 and was proven to be correct by restriction-enzyme analysis and DNA-sequencing according to the Sanger method (1977).

All cloning steps were essentially carried out as described by Maniatis et al. (1992). The plasmid pGBT-VP3 was prurified by centrifugation in a CsCl gradient and column chromatography in Sephacryl S500 (Pharmacia).

### GAL4-activation domain-tagged cDNA library

The expression vector pACT, containing the cDNAs from Epstein-Barr-virus-transformed human B cells fused to the GAL4 transcriptional activation domain, was used for detecting apoptin-associating proteins. The pACT c-DNA lbrary is derived from the lambda-ACT cDNA library, as described by Durfee et al. 1993.

### Bacterial and Yeast strains

The E.coli strain JM109 was the transformation recipient for the plasmid pGBT9 and pGBT-VP3. The bacterial strain electromax/DH10B was used for the transformation needed for the recovery the apoptin-associating pACT-cDNAs, and was obtained from GIBCO-BRL, USA.

The yeast strain Y190 was used for screening the cDNA library, and all other transformations which are part of the used yeast-two-hybrid system.

### Media

For drug selections Luria Broth (LB) plates for E.coli were supplemented with ampicillin (50 microgram per ml). Yeast YPD and SC media were prepared as described by Rose et al. (1990).

### Transformation of competent yeast strain Y190 with plasmids pGBT-VP3 and pACT-cDNA and screening for beta-galactosidase activity.

The yeast strain Y190 was made competent and transformed according to the methods described by Klebe et al. (Klebe et al., 1983). The yeast cells were first transformed with pGBT-VP3 and subsequently transformed with pACT-cDNA, and these transformed yeast cells were grown on histidine-minus plates, also lacking leucine and tryptophan.

Hybond-N filters were layed on yeast colonies , which were histidine-positive and allowed to wet completely. The filters were lifted and submerged in lquid nitrogen to permeabilize the yeast cells. The filters were thawed and layed with the colony side up on Whattman 3MM paper in a petridish with Z-buffer (Per liter: 16.1 gr Na₂HPO₄.7H₂O, 5.5 gr NaH₂PO₄.H₂O, 0.75 gr KCl and 0,246 gr MgSO₄.7H₂O, pH 7.0) containing 0.27% beta-mercapto-ethanol and 1 mg/ml X-gal. The filters were incubated for at least 15 minutes or during night.

### Recovery of plasmids from yeast

Total DNA from yeast cells, which were histidine- and beta-galactosidase-positive, was prepared by using the glusulase-alkaline lysis method as described by Hoffman and Winston (1987) and used to transform Electromax/DH10B bacteria via electroporation using a Bio-Rad GenePulser according the manufacturer's specifications.

Transformants were plated on LB media containing ampicillin.

### Isolation of apoptin-associating pACT clones

By means of colony-filter assay the colonies were lysed and hybridized to a radioactive-labeled 17-mer oligomer, which is specific for pACT (see also section Sequence analysis).

Plasmid DNA was isolated from the pACT-clones, and by means of XhoI digestion analysed for the presence of a cDNA insert.

### Sequence analysis

The subclones containing the sequences encoding apoptin-associating proteins were sequenced using dideoxy NTPs according to the Sanger method which was performed by Eurogentec, Nederland BV (Maastricht, The Netherlands). The used sequencing primer was a pACT-specific 17-mer comprising of the DNA-sequence 5'-TACCACTACAATGGATG-3'.

The sequences of the apoptin-associating proteins were compared with known gene sequences from the EMBL/Genbank.

### Results and discussion

Apoptin induces specifically apoptosis in transformed cells, such as cell lines derived from human tumors. To identify the essential compounds in this cell-transformation-specific and/or tumor-specific apoptosis pathway, a yeast genetic screen was carried out.

We have used a human cDNA library, which is based on the plasmid vector pACT containing the complete cDNA copies made from Epstein-Barr virus-transformed human B cells (Durfee et al., 1993).

### Construction of a bait plasmid expressing a fusion gene product of GAL4-DNA-binding domain and apoptin

To examine the existence of apoptin-associating proteins in the human transformed/tumorigenic cDNA library, a so-called bait plasmid had to be constructed.

To that end, the complete apoptin-encoding region, flanked by about 40 basepairs downstream from the apoptin gene, was cloned in the multiple cloning site of plasmid pGBT9.

The final construct, called pGBT-VP3, was analysed by restriction-enzyme analysis and sequencing of the fusion area between apoptin and the GAL4-DNA-binding domain.

### A gene(fragment) encoding an apoptin-associating protein is determined by transactivation of a GAL4-responsive promoter in yeast.

The apoptin gene is fused to the GAL4-DNA-binding domain of plasmid pGBT-VP3, whereas all cDNAs derived from the transformed human B cells are fused to the GAL4-activation domain of plasmid pACT. If one of the cDNAs will bind to apoptin, the GAL4-DNA-binding domain will be in the vicinity of the GAL4-activation domain resulting in the activation of the GAL4-responsive promoter, which regulates the reporter genes HIS3 and LacZ.

The yeast clones containing plasmid expressing apoptin and a plasmid expressing an apoptin-associating protein fragment can grow on a histidine-minus medium and will stain blue in a beta-galactosidase assay. Subsequently, the plasmid with the cDNA insert encoding the apoptin-associating protein can be isolated and characterized.

Before we could do so, however, we have determined that transformation of yeast cells with pGBT-VP3 plasmid alone or in combination with an empty pACT vector, did not result in the activation of the GAL4-responsive promoter.

### Identification of apoptin-associating proteins encoded by cDNAs derived from a human transformed B cell line.

We have found yeast colonies, which upon transformation with pGBT-VP3 and pACT-cDNA were able to grow on a histidine-minus medium (also lacking leucine and tryptophan) and stained blue in a beta-galactosidase assay. These results indicate that the observed yeast colonies contain besides the bait plasmid pGBT-VP3 also a pACT plasmid encoding a potential apoptin-associating protein.

Plasmid DNA was isolated from these positive yeast colonies, which were transformed in bacteria. By means of a filter-hybridization assay using a pACT-specific labeled DNA-probe, the clones containing pACT plasmid could be determined. Subsequently, pACT DNA was isolated and digested with restriction enzyme XhoI, which is indicative for the presence of a cDNA insert. Finally, the pACT plasmids with a cDNA insert were sequenced.

### Description of apoptin-associating proteins

The yeast genetic screen for apoptin-associating proteins resulted in the detection of two types of proteins, namely a Hou/Nmi-like protein and an IFP35-like protein. The apoptin-associating amino-acid sequences are homologous with the known Hou/Nmi amino-acid sequence or homologous with the known IFP35 amino-acid sequence. Hou/Nmi also share a homologous region (see below).

The determined DNA sequences of the two independent Hou/Nmi cDNA clones are shown in Figures 1 and 2, respectively. The amino acid sequence, derived from the detected DNA sequences is given in Figure 3. Remarkably, the complete open-reading frame (ORF) of the Hou-like protein was proven to be characterized.

The found DNA sequences of the three independent IFP35-like cDNA clones are shown in Figures 4, 5 and 6, respectively. Figure 7 shows the combination of the 2 independent IFP35 amino-acid sequences. The common part of these clones will associate with apoptin.

Interestingly, the C-terminus of Nmi shows homology to IFP35 (a.a. 102-288, 46% similarity; (Bao and Zervos, 1996, Bange et al, 1994). Actually, these data show that it is expected that our genetic yeast screen has resulted in these two apoptin-associating proteins, for they share a common homologous region.

### Hou/Nmi-like proteins

The remarkable feature of apoptin-induced apoptosis is its tumor-specific activity. The fact that apoptin binds to Hou/Nmi-like proteins unravels this tumor/transformation-specific activity of apoptin. Below, the terms Hou/Nmi-like, Nmi, or Hou will be interchangeably used.

In this respect, the pattern of Nmi expression is interesting, since it is expressed at low levels in normal tissues, in contrast to its high levels of expression in transformed cell lines. Among eight cancer lines tested, highest levels were observed in four leukemia cell lines (Bao and Zervos, 1996).

In leukemias, a high expression of C-myc correlates with a high level of Nmi (HL-60, K562 and MOLT-4). The Nmi gene is located on chromosome 22, which is also involved in the t (9;22) translocation leading to the Bcr-Abl fusion protein, as seen in some leukemias (Rabbits, 1991, Sawyers and Deny, 1994).

Using a yeast genetic screen, Nmi was identified as a protein that binds to N-myc and C-myc. Myc proteins are important in the regulation of cell proliferation and differentiation. Together with ras or raf, myc can transform primary cells in culture. Nmi/Hou-like proteins will up-regulate the activity of Myc proteins via binding to them.

Up-regulation of Myc proteins has been described for Burkitt lymphomas, neuroblastomas and small cell lung carcinomas. Myc proteins contain a basic region, a helix-loop-helix (HLH) and a leucine zipper (Zip), and form homo-or heterodimers that can bind to specific DNA sequences and regulate transcription. Myc also forms heterodimers with Max. Myc/Max heterodimers activate transcription, whereas Max homodimers repress transcription, thus antagonizing Myc's function (Evan and Littlewood, 1993).

Nmi was found to interact with N-myc, c-myc, Max, Mxi1 and other transcription factors that have HLH and/or Zip motifs. Interaction with N-myc and C-myc was confirmed by co-precipitation experiments (Bao and Zervos, 1996).

### Induction of apoptosis through interference with the function of Nmi/Hou-like proteins.

Our results indicate that apoptin can change the Nmi/Hou-like-mediated proliferation (transformation/tumor-formation) activity into a Nmi/Hou-like-mediated apoptotic activity. Remarkably, this Nmi/Hou-like-mediated apoptotic activity will be specific for transformed/tumor cells, due to the very high level of Nmi/Hou in transformed cells in combination with over-expression of (proto-)oncogenes, such as Myc.

By means of transient transfection assays, it was shown that over-expression of the determined Hou-like protein (see Fig. 3) and apoptin did result in induction of apoptosis in normal VH10-, VH25-fibroblasts. In contrast to normal fibroblasts which over-expressed only apoptin. This result indicates that Hou-like proteins are an important factor in (apoptin-induced) apoptosis.

The presented data imply that interference with the function of Nmi/Hou-like proteins resulting in apoptosis can be used as a specific anti-tumor therapy, or therapies of related diseases, such as auto-immune diseases.

### Characteristics of the apoptin-associating protein IFP35

The other apoptin-associating protein is IFP35, which is an interferon(IFN)-induced leucine zipper protein of 282 a.a., and has an apparent molecular mass of 35 kD. It was isolated by differential screening from HeLa cells that had been treated with IFN-γ (Bange et al., 1994).

IFP35 mRNA could be induced by IFN-γ in different human cell types, including fibroblasts, macrophages, and epithelial cells. It has a leucine zipper motif at the N-terminus, but it lacks an adjacent basic domain required for DNA binding. It has been suggested that these types of proteins negatively regulate bZIP transcription factors by forming non-functional heterodimers. IFP35 was shown to form homodimers (Bange et al., 1994).

### Induction of apoptosis by interference of IFP35 in combination with Hou/Nmi-like proteins.

IFP35 is found in the cell nucleus, after interferon treatment and is expressed in a wide variety of cell types including fibroblasts, macrophages and epithelial cells (Bange et al., 1994).

In general, virus infections trigger interferon production. It is likely that a CAV infection and/or expression of apoptin will result in interferon up-regulation, which might result in the translocation of IFP35 or IFP35-like proteins into the nucleus. IFP35 will transport apoptin also to the nucleus, due to its association.

It seems likely that if apoptin is transported into the nucleus by IFP35 it will be able to associate with the IFP35-homologous region within Hou/Nmi-like proteins. This association will cause an aberrant regulation of Hou/Nmi-regulated genes, such as the oncogene Myc. Subsequently, the cells over-expressing Nmi/Hou-like proteins and oncogenes, such as Myc will undergo apoptosis.

Experimental evidence for IFP35 as an essential factor in (apoptin) apoptosis induction was derived from the following experiments. Normal VH10 cells over-expressing Hou/Nmi, IFP35 and apoptin underwent faster apoptosis than normal VH10 cells expressing Hou/Nmi and apoptin.

### Conclusions

In conclusion, we have provided evidence that interference of specific factors with the function of Nmi/Hou, of IFP35 or of both will result in induction of apoptosis.

Therapies based on induction of apoptosis are possible if they succeed in the interference with the function of Nmi/Hou-like and/or of IFP35-like proteins. An example of such an interfering factor is apoptin. Another CAV-derived protein, which is known to induce apoptosis and also known to enhance apoptin activity is VP2 (Noteborn et al., 1997).

### Other apoptin-associating proteins

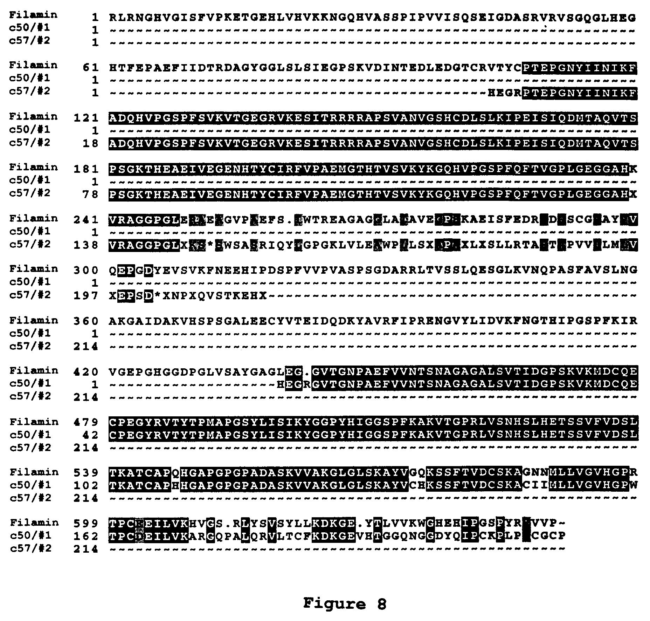

The genetic yeast screen with pGBT-VP3 as bait plasmid and pACT plasmid containing cDNAs from transformed human B cells also delivered the protein filamin. The protein filamin is localized within lamellipodia and filopodia. Filamin is one of the cross-linking proteins of actin. It may play an additional role of linking the cytoskeleton to cell-substratum adhesion sites (Matsudaira, 1994).
Two independent filamin-like clones were found. The found associating amino acid sequence of the two filamin clones are shown in Figure 8.

### Description of the figures

Figure 1 shows the DNA sequence of the analysed region of the apoptin-associating clone Nmi/Hou-like No-1.
Figure 2 shows the DNA sequence of the analysed region of the apoptin-associating clone Nmi/Hou-like No-2.
Figure 3 shows the combination of the amino acids of the sequenced Nmi/Hou-like, derived from clones No-1 and No-2. In addition, the three C-terminal amino acids H-E-G of the multiple cloning site of pACT are given to illustrate that the Nmi/Hou-like amino acid sequence is in frame with the GAL4-activation domain. This feature proves that the Nmi/Hou-like region is indeed synthesized in yeast cells.
Figure 4 shows the DNA sequence of the analysed region of the apoptin-associating clone IFP35-like No-1.
Figure 5 shows the DNA sequence of the analysed region of the apoptin-associating clone IFP35-like No-2.
Figure 6 shows the amino acids of the sequenced region of the apoptin-associating clone IFP35-like No-3.
Figure 7 shows the combination of the amino acids of the sequenced IFP35-like clones No-2 and No-3. The fact that they overlap with each other implies that the common region of these three inserts associates with apoptin. In addition, part of the amino acid sequence of the known IFP35 is shown.
Figure 8 shows the amino acids of the sequenced region of the apoptin-associating clone Filamin No-1 and No-2. In addition, the three C-terminal amino acids H-E-G of the multiple cloning site of pACT are given to illustrate that the filamin-like amino acid sequence is in frame with the GAL4-activation domain. This feature proves that the filamin-like region is indeed synthesized.

### REFERENCES

1. Bange, F.C., Vogel, U., Flohr, T., Kiekenbeck, M., Denecke, B., and Boettger, E.C. (1994). IFP35 is an interferon-induced leucine zipper protein that indergoes interferon-regulated cellular redistribution. The Journal of Biological Chemistry 269, 1091-1098.
2. Bao, J. and Zervos, A.S. (1996) Isolation and characterization of Nmi, a novel partner of Myc proteins. Oncogene 12, 2171-2176.
3. Bellamy, C.O.C., Malcomson, R.D.G., Harrison, D.J., and Wyllie, H. 1995. Cell death and disease: The biology and regulation of apoptosis. Seminars in Cancer Biology 6, 3-12.
4. Danen-Van Oorschot, A.A.A.M., Fischer, D.F., Grimbergen, J.M., Klein, B., Zhuang, S.-M., Falkenburg, J.H.F., Backendorf, C., Quax, P.H.A., Van der Eb, J.A., and Noteborn, M.H.M. (1997). Apoptin induces apoptosis in human transformed and malignant cells but not in normal cells. Proceedings National Academy Sciences, USA: 94, 5843-5847.
5. Danen-Van Oorschot, A.A.A.M, Den Hollander, A., Takayama, S., Reed, J., Van der Eb, A.J. and Noteborn, M.H.M. (1997a). BAG-1 inhibits p53-induced but not apoptin-induced apoptosis. Apoptosis 2, 395-402.
6. Duke, R.C., Ocjius, D.M., Young, J, D-E. (1996). Cell suicide in health and disease. Scientific American December 1996, 48-55.
7. Durfee, T., Becherer, K., Chen, P.-L., Yeh,S.-H., Yang, Y., Kilburn, A.E., Lee, W.-H., and Elledge, S.J. (1993). The retinoblastoma protein associates with the protein phosphate type I catalytic subunit. Genes and Development 7, 555-569.
8. Earnshaw, W.C., 1995. Nuclear changes in apoptosis. Current Opinion in Cell Biology 7, 337-343.
9. Evan, G.I. and Littlewood, T.D. (1993). Current Opinions in Genetics Development 3, 44-49.
10. Fields, S. and Song, O.K. (1989). A novel genetic system to detect protein-protein interactions. Nature 340, 245-246.
11. Hockenberry, D.M. (1994). Bcl-2 in cancer, development and apoptosis. Journal of Cell Science, Supplement 18, 51-55.
12. Hoffman, C.S. and Winston, F. (1987). A ten-minute DNA preparation from yeast efficiently releases autonomous plasmids for transformation of Escherichia coili. Gene 57, 267-272.
13. Kerr, J.F.R., Winterford, C.M., and Harmon, B.V. (1994). Apoptosis: Its significance in cancer and cancer therapy. Cancer 73, 2013-2026.
14. Klebe, R.J., Harriss, J.V., Sharp, Z.D., and Douglas, M.G. (1983). A general method for polyethylene-glycol-induced genetic transformation of bacteria and yeast. Gene 25, 333-341.
15. Levine, A.J. (1997). p53, the cellular gatekeeper for growth and division. Cell 88, 323-331.
16. Maniatis, T., Fritsch, E.F., and Sambrook, J. (1982). Molecular Cloning: A Laboratory Manual. CSHL Press, New York, USA.
17. Matsudaira, P. (1994). Actin crosslinking proteins at the leading edge. Seminars in Cell biology 5, 165-174.
18. McDonell T.J., Meyn, R.E., Robertson, L.E. (1995). Implications of apoptotic cell death regulation in cancer therapy. Seminars in Cancer Biology 6, 53-60.
19. Noteborn, M.H.M. (1996). PCT application WO 96/41191. Apoptin induces apoptosis in human transformed and malignant cells but not in normal cells as essential characteristic for the development of an anti-tumor therapy.
20. Noteborn, M.H.M., and De Boer, G.F. (1996). Patent USA/no. 030, 335.
21. Noteborn, M.H.M., De Boer, G.F., Van Roozelaar, D., Karreman, C., Kranenburg, O., Vos, J., Jeurissen, S., Zantema, A., Hoeben, R., Koch, G., Van Ormondt, H., and Van der Eb, A.J. (1991). Characterization of cloned chicken anemia virus DNA that contains all elements for the infectious replication cycle. Journal of Virology 65, 3131-3139.
22. Noteborn, M.H.M., Hoeben, R.C., and Pietersen, A. (1997). A gene delivery vehicle expressing the apoptosis-inducing proteins VP2 and/or apoptin. European Patent Application no. 97201121.7
23. Noteborn, M.H.M., Todd, D., Verschueren, C.A.J., De Gauw, H.W.F.M., Curran, W.L., Veldkamp, S., Douglas, A.J., McNulty, M.S., Van der Eb, A.J., and Koch, G. (1994). A single chicken anemia virus protein induces apoptosis. Journal of Virology 68, 346-351.
24. Noteborn, M.H.M., and Zhang, Y. (1997). Methods and means for determining the transforming capability of agents, for determining the predisposition of cells to become transformed and prophylactic treatment of cancer using apoptin-like activity. European Patent Application no. 97439
25. Paulovich, A.G., Toczyski, D., Hartwell, H. (1997). When checkpoints fail. Cell 88, 315-321.
26. Rabbitts, T.H. (1991). Cell 67, 641-644.
27. Rose, M.D., Winston, F., and Hieter, P. (1990). Methods in yeast genetics. A laboratory course manual. Cold Spring Harbor Laboratory Press, Cold Spring Harbor, New York, USA.
28. Sachs, L. and Lotem, J. (1993). Control of programmed cell death in normal and leukemia cells: New implications for therapy. Blood 82, 15-21.
29. Sanger, F., Nicklen, S., and Coulsen, A.R. (1977). DNA sequencing with chain-terminating inhibitors. Proceedings National Academic Sciences USA 74, 5463-5467.
30. Steller, H. (1995). Mechanisms and genes of cellular suicide. Science 267, 1445-1449.
31. Sawyers, C.L. and Denny, C.T. (1994) Cell 77, 171-173.
32. Telford, W.G., King, L.E., Fraker, P.J. (1992). Comparative evaluation of several DNA binding dyes in the detection of apoptosis-associated chromatin degradation by flow cytometry. Cytometry 13, 137-143.
33. Teodoro, J.G. and Branton, P.E. (1997). Regulation of apoptosis by viral gene products. Journal of Virology 71, 1739-1746.
34. Thompson, C.B. (1995). Apoptosis in the pathogenesis and treatment of disease. Science 267, 1456-1462.
35. White, E. (1996). Life, death, and the pursuit of apoptosis. Genes and development 10, 1-15.
36. Wyllie, A.H. (1995). The genetic regulation of apoptosis. Current Opinion in Genetics and Development 5, 97-104.
37. Wyllie, A.H., Kerr, J.F.R., Currie, A.R. (1980). Cell death: The significance of apoptosis. International Review of Cytology 68, 251-306.
38. Yang, X., Hubbard, E.J.A., and Carlson, M. (1992). A protein kinase substrate identified by the two-hybrid system. Science 257, 680-682.
39. Zhuang, S.-M., Landegent, J.E., Verschueren, C.A.J., Falkenburg, J.H.F., Van Ormondt, H., Van der Eb, A.J., Noteborn, M.H.M. (1995). Apoptin, a protein encoded by chicken anemia virus, induces cell death in various human hematologic malignant cells in vitro. Leukemia 9 S1, 118-120.
40. Zhuang, S.-M., Shvarts, A., Van Ormondt, H., Jochemsen, A.-G., Van der Eb, A.J., Noteborn, M.H.M. (1995). Apoptin, a protein derived from chicken anemia virus, induces a p53-independent apoptosis in human osteosarcoma cells. Cancer Research 55, 486-489.

SEQUENCE LISTING

(1) GENERAL INFORMATION:

(i) APPLICANT:
(A) NAME: Leadd B.V.
(B) STREET: Wassenaarseweg 72
(C) CITY: Leiden
(D) STATE: Zuid-Holland
(E) COUNTRY: the Netherlands
(F) POSTAL CODE (ZIP): 2333 AL

(ii) TITLE OF INVENTION: Novel molecules involved in
apoptotic
pathways.

(iii) NUMBER OF SEQUENCES: 14

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version
#1.30 (EPO)

(v) CURRENT APPLICATION DATA:
APPLICATION NUMBER: EP 97203781.6

(2) INFORMATION FOR SEQ ID NO: 1:

(i) SEQUENCE CHARACTERISTICS:
(A) LENGTH: 17 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 1:

TACCACTACA ATGGATG
17

(2) INFORMATION FOR SEQ ID NO: 2:

(i) SEQUENCE CHARACTERISTICS:
(A) LENGTH: 658 base pairs
(B) TYPE: nucleic acid
(C) STRANDEDNESS: unknown
(D) TOPOLOGY: unknown

(ii) MOLECULE TYPE: DNA (genomic)

(iii) HYPOTHETICAL: NO

(ix) FEATURE:
(A) NAME/KEY: misc_feature
(B) LOCATION:579..658
(D) OTHER INFORMATION:/label= N
/note= ""N" stands for unknown."

(xi) SEQUENCE DESCRIPTION: SEQ ID NO: 2:

GGGGGATCAT GGAAGCTGAT AAAGATGACA CACAACAAAT TCTTAAGGAG
CATTCGCCAG 60

ATGAATTTAT AAAAGATGAA CAAAATAAGG GACTAATTGA TGAAATTACA
AAGAAAAATA 120

TTCAACTAAA GAAGGAGATC CAAAAGCTTG AAACGGAGTT ACAAGAGGCT
ACCAAAGAAT 180

TCCAGATTAA AGAGGATATT CCTGAAACAA AGATGAAATT CTTATCAGTT
GAAACTCCTG 240

AGAATGACAG CCAGTTGTCA AATATCTCCT GTTCGTTTCA AGTGAGCTCG
AAAGTTCCTT 300

ATGAGATACA AAAAGGACAA GCACTTATCA CCTTTGAAAA AGAAGAAGTT
GCTCAAAATG 360

TGGTAAGCAT GAGTAAACAT CATGTACAGA TAAAAGATGT AAATCTGGAG
GTTACGGCCA 420

AGCCAGTTCC ATTAAATTCA GGAGTCAGAT TCCAGGTTTA TGTAGAAGTT
TCTAAAATGA 480

AAATCAATGT TACTGAAATT CCTGACACAT TGCGTGAAGA TCAAATGAGA
GACAAACTAG 540

AGCTGAGCTT TTCAAAGTCC CGAAATGGGA GGCGGAGANG TGGACCGCGT
GGGACTATGA 600

CAGACAGTCC GGGAGTGCAG TCATCACGTT TGGNGGAGAT TGGGAGTGGC
TGACANNN 658

(2) INFORMATION FOR SEQ ID NO: 3:

(i) SEQUENCE CHARACTERISTICS:
(A) LENGTH: 719 base pairs
(B) TYPE: nucleic acid
(C) STRANDEDNESS: unknown
(D) TOPOLOGY: unknown

(ii) MOLECULE TYPE: DNA (genomic)

(iii) HYPOTHETICAL: NO

(ix) FEATURE:
(A) NAME/KEY: misc_feature
(B) LOCATION:89..716
(D) OTHER INFORMATION:/label= N
/note= ""N" stands for unknown."

(xi) SEQUENCE DESCRIPTION: SEQ ID NO: 3:

CGGAGTTACA AGAGGCTACC AAAGAATTCC AGATTAAAGA GGATATTCCT
GAAACAAAGA 60

TGAAATTCTT ATCAGTTGAA ACTCCTGANA ATGACAGCCA GTTGTCAAAT
ATCTCCTGTT 120

CGTTTCAAGG TGAGCTCGAA AGTTCCTTAT GAGATACAAA AAGGACAATG
CACTTATCAC 180

CTTTGAAAAA GGAAGAAGTT GCTCAAAATG TGNGTAANGC ATGAGTAAAC
ATCATGTACA 240

GATAATAAGA TGTAAATCTG GAGGTTACGG CCAAAGCCAA GTTCCATTAA
TATTCAAGGA 300

GTCANGATTC CAGNGTTTAT GCTAGAANGT TTCTAAAAAT GANAATCAAT
GGTTACTGGA 360

AATTCCTGGA CACATTGCGN TGAAAGATCA AGATGACGAA GACAAACTAA
GAAGCTGAGC 420

TTTTCAAAAG TCCCGAAANA TGGAAGAGCG GTAGAGGGTG GNACCGCGTG
NGANCTATGA 480

CAAGACAAGN CCGGGGAAGN TGCAGTCCAT CACGTTTGTN NGAAGATTGG
ANGTNGGCTG 540

ACCAANGAAT TTTGAAAAAG GAGANGAATT ACCCCTCTTT ANGAGTAANA
TCAAAACCCT 600

GCCATAANAA GTTNACTGGT TTCNCCCATT ACACAGNANT TACANNTTGA
NCAANANTAN 660

NCAGGATAAT TTNCAGGGGA ANAATCTNAA GNATGGCAAG NTGACTTCTG
GACAANGGT 719

(2) INFORMATION FOR SEQ ID NO: 4:

(i) SEQUENCE CHARACTERISTICS:
(A) LENGTH: 220 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 4:

His Glu Gly Arg Gly Ile Met Glu Ala Asp Lys Asp Asp
Thr Gln Gln
1               5                   10
15

Ile Leu Lys Glu His Ser Pro Asp Glu Phe Ile Lys Asp
Glu Gln Asn
                20                  25
30

Lys Gly Leu Ile Asp Glu Ile Thr Lys Lys Asn Ile Gln
Leu Lys Lys
            35                  40                  45

Glu Ile Gln Lys Leu Glu Thr Glu Leu Gln Glu Ala Thr
Lys Glu Phe
        50                  55                  60

Gln Ile Lys Glu Asp Ile Pro Glu Thr Lys Met Lys Phe
Leu Ser Val
65                  70                  75
80

Glu Thr Pro Glu Asn Asp Ser Gln Leu Ser Asn Ile Ser
Cys Ser Phe
                85                  90
95

Gln Val Ser Ser Lys Val Pro Tyr Glu Ile Gln Lys Gly
Gln Ala Leu
                100                 105
110

Ile Thr Phe Glu Lys Glu Glu Val Ala Gln Asn Val Val
Ser Met Ser
            115                 120                 125

Lys His His Val Gln Ile Lys Asp Val Asn Leu Glu Val
Thr Ala Lys
        130                 135                 140

Pro Val Pro Leu Asn Ser Gly Val Arg Phe Gln Val Tyr
Val Glu Val
145                 150                 155
160

Ser Lys Met Lys Ile Asn Val Thr Glu Ile Pro Asp Thr
Leu Arg Glu
                165                 170
175

Asp Gln Met Arg Asp Lys Leu Glu Leu Ser Phe Ser Lys
Ser Arg Asn

180 185
190

Gly Arg Arg Arg Cys Gly Pro Arg Gly Thr Met Thr Asp
Ser Pro Gly
195 200 205

Val Gln Ser Ser Arg Leu Val Glu Ile Gly Ser Gly
    210                 215                 220

(2) INFORMATION FOR SEQ ID NO: 5:

(i) SEQUENCE CHARACTERISTICS:
(A) LENGTH: 307 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 5:

Met Glu Ala Asp Lys Asp Asp Thr Gln Gln Ile Leu Lys
Glu His Ser
1 5 10
15

Pro Asp Glu Phe Ile Lys Asp Glu Gln Asn Lys Gly Leu
Ile Asp Glu
20 25
30

Ile Thr Lys Lys Asn Ile Gln Leu Lys Lys Glu Ile Gln
Lys Leu Glu
35 40 45

Thr Glu Leu Gln Glu Ala Thr Lys Glu Phe Gln Ile Lys
Glu Asp Ile
50 55 60

Pro Glu Thr Lys Met Lys Phe Leu Ser Val Glu Thr Pro
Glu Asn Asp
65 70 75
80

Ser Gln Leu Ser Asn Ile Ser Cys Ser Phe Gln Val Ser
Ser Lys Val
85 90
95

Pro Tyr Glu Ile Gln Lys Gly Gln Ala Leu Ile Thr Phe
Glu Lys Glu

100 105
110

Glu Val Ala Gln Asn Val Val Ser Met Ser Lys His His
Val Gln Ile
115 120 125

Lys Asp Val Asn Leu Glu Val Thr Ala Lys Pro Val Pro
Leu Asn Ser
130 135 140

Gly Val Arg Phe Gln Val Tyr Val Glu Val Ser Lys Met
Lys Ile Asn
145 150 155
160

Val Thr Glu Ile Pro Asp Thr Leu Arg Glu Asp Gln Met
Arg Asp Lys
165 170
175

Leu Glu Leu Ser Phe Ser Lys Phe Arg Asn Gly Gly Gly
Glu Val Asp
180 185
190

Arg Val Asp Tyr Asp Arg Gln Ser Gly Ser Ala Val Ile
Thr Phe Val
195 200 205

Glu Ile Gly Val Ala Asp Lys Ile Leu Lys Lys Lys Glu
Tyr Pro Leu
210 215 220

Tyr Ile Asn Gln Thr Cys His Arg Val Thr Val Ser Pro
Tyr Thr Glu
225 230 235
240

Ile His Leu Lys Lys Tyr Gln Ile Phe Ser Gly Thr Ser
Lys Arg Thr
245 250
255

Val Leu Leu Thr Gly Met Glu Gly Ile Gln Met Asp Glu
Glu Ile Val
260 265
270

Glu Asp Leu Ile Asn Ile His Phe Gln Arg Ala Lys Asn
Gly Gly Gly
275 280 285

Glu Val Asp Val Val Lys Cys Ser Leu Gly Gln Pro His
Ile Ala Tyr
290 295 300

Phe Glu Glu

305

(2) INFORMATION FOR SEQ ID NO: 6:

(i) SEQUENCE CHARACTERISTICS:
(A) LENGTH: 659 base pairs
(B) TYPE: nucleic acid
(C) STRANDEDNESS: unknown
(D) TOPOLOGY: unknown

(ii) MOLECULE TYPE: DNA (genomic)

(iii) HYPOTHETICAL: NO

(ix) FEATURE:
(A) NAME/KEY: misc_feature
(B) LOCATION:189..657
(D) OTHER INFORMATION:/label= N
/note= ""N" stands for unknown."

(xi) SEQUENCE DESCRIPTION: SEQ ID NO: 6:

AGCAGGTGCT GCAACAAAAG GAGCACACGA TCAACATGGA GGAGTGCCGG
CTGCGGGTGC 60

AGGTCCAGCC CTTGGAGCTG CCCATGGTCA CCACCATCCA GGTGTCCAGC
CAGTTGAGTG 120

GCCGGAGGGT GTTGGTCACT GGATTTCCTG CCAGCCTCAG GCTGAGTGAG
GAGGAGCTGC 180

TGGACAANCT ANAGATCTTC TTTGGCAAGA CTAGGAACGG AGGTGGCNAT
GTGGACNTTC 240

GGGANCTACT GCCAGGGANT GTCATGCTGG GGTTTGCTAG GGATGGAGTG
GCTCANCGTC 300

TGTGCCAAAT CGGCCATTTC ACAGTGCCAC TGGGTGGGCA GCANGTCCCT
CTGAGAGTCT 360

CTCCGTATGT GAATGGGGAN ATCCAGANGG CTGANATCAG GTCNCAGCCA
NTTCCCCGCT 420

CGGTACTGGT GCTCAACATT CCTGATATCT TGGATGGCCC GGAGCTGCAT
GACGTCCTGG 480

ANATCCACTT CCAGAANCCC ACCCGCGGGG GCGGAGATGT AAGACGCCCT
GACAGTCGTA 540

CCCCAAGGAC AACAGGGCCT AACAGTCTTC ACCTCCTGAA TCAAGGCTAN
GGGCCTCCCC 600

CTTCTCATCC TCCCCACCCC CCCCGCCAAA GGTTCTCAAN ACTGGGCCTG
GGCTTTNTG 659

(2) INFORMATION FOR SEQ ID NO: 7:

(i) SEQUENCE CHARACTERISTICS:
(A) LENGTH: 630 base pairs
(B) TYPE: nucleic acid
(C) STRANDEDNESS: unknown
(D) TOPOLOGY: unknown

(ii) MOLECULE TYPE: DNA (genomic)

(iii) HYPOTHETICAL: NO

(ix) FEATURE:
(A) NAME/KEY: misc_feature
(B) LOCATION:354..621
(D) OTHER INFORMATION:/label= N
/note= ""N" stands for unknown."

(xi) SEQUENCE DESCRIPTION: SEQ ID NO: 7:

CCAAAGTGGC TGAGCAGGTG CTGCAACAAA AGGAGCACAC GATCAACATG
GAGGAGTGCC 60

GGCTGCGGGT GCAGGTCCAG CCCTTGGAGC TGCCCATGGT CACCACCATC
CAGGTGTCCA 120

GCCAGTTGAG TGGCCGGAGG GTGTTGGTCA CTGGATTTCC TGCCAGCCTC
AGGCTGAGTG 180

AGGAGGAGCT GCTGGACAAG CTAGAGATCT TCTTTGGCAA GACTAGGAAC
GGAGGTGGCG 240

ATGTGGACGT TCGGGAGCTA CTGCCAGGGA GTGTCATGCT GGGGTTTGCT
AGGGATGGAG 300

TGGCTCAGCG TCTGTGCCAA ATCGGCCAAG TTCACAGTGC CACTGGGTGG
GCANCAAGTC 360

CCTCTGAGAG TCTCTCCGTA TGTGAATGGG GAGATCCAGA AGGCTGAGAT
CAGGTCGCAN 420

CCAGTTCCCC NCTCGGTACT GGGTGCTCAA CATTCCTGAT ATCTTGGATT
GGCCCGGAGC 480

TGCATNACGT CCTGGANATC AACTTCANAA GCCCACCCGC CGGGGCNGNG
AGGTANAAGG 540

CCTGACATCN TTACCCCAAA GGACAGCATG GNCCTAACAG TCCTCACCTC
CNAATCANGC 600

TNNGGGGCTN CCCTTCTANC NTCCCAACTG
630

(2) INFORMATION FOR SEQ ID NO: 8:

(i) SEQUENCE CHARACTERISTICS:
(A) LENGTH: 631 base pairs

(B) TYPE: nucleic acid
(C) STRANDEDNESS: unknown
(D) TOPOLOGY: unknown

(ii) MOLECULE TYPE: DNA (genomic)

(iii) HYPOTHETICAL: NO

(ix) FEATURE:
(A) NAME/KEY: misc_feature
(B) LOCATION:182..626
(D) OTHER INFORMATION:/label= N
/note= ""N" stands for unknown."

(xi) SEQUENCE DESCRIPTION: SEQ ID NO: 8:

GGATCCACTG CCCTCTGCTT GCGGGCTCTG CTCTGATCAC CTTTGATGAC
CCCAAAGTGG 60

CTGAGCAGGT GCTGCAACAA AAGGAGCACA CGATCAACAT GGAGGAGTGC
CGGCTGCGGG 120

TGCAGGTCCA GCCCTTGGAG CTGCCCATGG TCACCACCAT CCAGGTGATG
GTGTCCAGCC 180

ANTTGAGTGG CCGGAGGGTG TTGGTCACTG GATTTCCTGC CAGCCTCAGG
CTGANTGAGG 240

AGGAGCTGCT GGACAAGCTA TGAGATCTTC TTTGGCAANA CTANGAACGG
ANGTGGCGAT 300

GTGGACGTTC GGGAGCTACT GCCAGGGAGT GTCATGCTGG GGTTTGCTAC
GGATGGAGTG 360

GCTCAGCGTC TGTGCCAAAT CGGCCAGTTC ACAAGTGCCA CTGGGTGGGC
AGCAAGTCCC 420

TCTGAGAGTC TCTCCGTATG TGANTGGNGA GATCAGAATG CTGANATTAA
GTCGCATCCA 480

ATTCCTCGCT CNGGTACTGG TGCTCANNAT CCTGANATCT TGGATTGGCC
CCNGANTNCA 540

TGANATCTGG NAGATTCAAT TNCANAAGTC CANCCNNCNG NGNCGGGAAG
TANANGCCCG 600

ANANTTCNTN NCNTANGGNC AGCANNGCCT G
631

(2) INFORMATION FOR SEQ ID NO: 9:

(i) SEQUENCE CHARACTERISTICS:
(A) LENGTH: 138 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 9:

His Glu Gly Pro Lys Val Ala Glu Gln Val Leu Gln Gln
Lys Glu His
1               5                   10
15

Thr Ile Asn Met Glu Glu Cys Arg Leu Arg Val Gln Val
Gln Pro Leu
                20                  25
30

Glu Leu Pro Met Val Thr Thr Ile Gln Val Ser Ser Gln
Leu Ser Gly
            35                  40                  45

Arg Arg Val Leu Val Thr Gly Phe Pro Ala Ser Leu Arg
Leu Ser Glu
        50                  55                  60

Glu Glu Leu Leu Asp Lys Leu Glu Ile Phe Phe Gly Lys
Thr Arg Asn
65                  70                  75
80

Gly Gly Gly Asp Val Asp Val Arg Glu Leu Leu Pro Gly
Ser Val Met
                85                  90
95

Leu Gly Phe Ala Arg Asp Gly Val Ala Gln Arg Leu Cys
Gln Ile Gly
                100                 105
110

Gln Val His Ser Ala Thr Gly Trp Ala Ser Ser Pro Ser
Glu Ser Leu
            115                 120                 125

Ser Val Cys Glu Trp Gly Asp Pro Glu Gly
    130                 135

(2) INFORMATION FOR SEQ ID NO: 10:

(i) SEQUENCE CHARACTERISTICS:
    (A) LENGTH: 282 amino acids
    (B) TYPE: amino acid
    (C) STRANDEDNESS: unknown
    (D) TOPOLOGY: unknown

(ii) MOLECULE TYPE: protein

(iii) HYPOTHETICAL: NO

(xi) SEQUENCE DESCRIPTION: SEQ ID NO: 10:

Met Ser Ala Pro Leu Asp Ala Ala Leu His Ala Leu Gln
Glu Glu Gln
1               5                   10
15

Ala Arg Leu Lys Met Arg Leu Trp Asp Leu Gln Gln Leu
Arg Lys Glu
                20                  25
30

Leu Gly Asp Ser Pro Lys Asp Lys Val Pro Phe Ser Val
Pro Lys Ile
            35                  40                  45

Pro Leu Val Phe Arg Gly His Thr Gln Gln Asp Pro Glu
Val Pro Lys
        50                  55                  60

Ser Leu Val Ser Asn Leu Arg Ile His Cys Pro Leu Leu
Ala Gly Ser
65                  70                  75
80

Ala Leu Ile Thr Phe Asp Asp Pro Lys Val Ala Glu Gln
Val Leu Gln
                    85                  90
95

Gln Lys Glu His Thr Ile Asn Met Glu Glu Cys Arg Leu
Arg Val Gln
                100                 105
110

Val Gln Pro Leu Glu Leu Pro Met Val Thr Thr Ile Gln
Val Ser Ser
            115                 120                 125

Gln Leu Ser Gly Arg Arg Val Leu Val Thr Gly Phe Pro
Ala Ser Leu
        130                 135                 140

Arg Leu Ser Glu Glu Glu Leu Leu Asp Lys Leu Glu Ile
Phe Phe Gly
145                 150                 155
160

Lys Thr Arg Asn Gly Gly Gly Asp Val Asp Val Arg Glu
Leu Leu Pro

165 170
175

Gly Ser Val Met Leu Gly Phe Ala Arg Asp Gly Val Ala
Gln Arg Leu
180 185
190

Cys Gln Ile Gly Gln Phe Thr Val Pro Leu Gly Gly Gln
Gln Val Pro
195 200 205

Leu Arg Val Ser Pro Tyr Val Asn Gly Glu Ile Gln Lys
Ala Glu Ile
210 215 220

Arg Ser Gln Pro Val Pro Arg Ser Val Leu Val Leu Asn
Ile Pro Asp
225 230 235
240

Ile Leu Asp Gly Pro Glu Leu His Asp Val Leu Glu Ile
His Phe Gln
245 250
255

Lys Pro Thr Arg Gly Gly Gly Gly Arg Gly Pro Asp Ser
Arg Thr Pro
260 265
270

Arg Thr Ala Gly Pro Ser Ser Leu His Leu
        275                 280

(2) INFORMATION FOR SEQ ID NO: 11:

(i) SEQUENCE CHARACTERISTICS:
(A) LENGTH: 207 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 11:

His Glu Gly Arg Ile His Cys Pro Leu Leu Ala Gly Ser
Ala Leu Ile
1 5 10
15

Thr Phe Asp Asp Pro Lys Val Ala Glu Gln Val Leu Gln
Gln Lys Glu

20                  25
30

His Thr Ile Asn Met Glu Glu Cys Arg Leu Arg Val Gln
Val Gln Pro
            35                  40                  45

Leu Glu Leu Pro Met Val Thr Thr Ile Gln Val Met Val
Ser Ser Xaa
        50                  55                  60

Leu Ser Gly Arg Arg Val Leu Val Thr Gly Phe Pro Ala
Ser Leu Arg
    65                  70                  75
80

Leu Xaa Glu Glu Glu Leu Leu Asp Lys Leu Asp Leu Leu
Trp Gln Xaa
                    85                  90
95

Xaa Glu Arg Xaa Trp Arg Cys Gly Arg Ser Gly Ala Thr
Ala Arg Glu
                100                 105
110

Cys His Ala Gly Val Cys Tyr Gly Trp Ser Gly Ser Ala
Ser Val Pro
            115                 120                 125

Asn Arg Pro Val His Lys Cys His Trp Val Gly Ser Lys
Ser Leu Glu
        130                 135                 140

Ser Leu Arg Met Xaa Xaa Arg Ser Glu Cys Xaa Val Ala
Ser Asn Ser
    145                 150                 155
160

Ser Leu Xaa Tyr Trp Cys Ser Xaa Ser Xaa Leu Gly Leu
Ala Pro Xaa
                    165                 170
175

Xaa Met Xaa Ser Gly Arg Phe Asn Xaa Xaa Ser Pro Xaa
Xaa Xaa Xaa
                180                 185
190

Gly Lys Xaa Xaa Pro Xaa Xaa Ser Xaa Xaa Xaa Xaa Ser
Xaa Ala
            195                 200                 205

(2) INFORMATION FOR SEQ ID NO: 12:

    (i) SEQUENCE CHARACTERISTICS:
        (A) LENGTH: 647 amino acids
        (B) TYPE: amino acid

(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 12:

Arg Leu Arg Asn Gly His Val Gly Ile Ser Phe Val Pro
Lys Glu Thr
1               5                   10
15

Gly Glu His Leu Val His Val Lys Lys Asn Gly Gln His
Val Ala Ser
                20                  25
30

Ser Pro Ile Pro Val Val Ile Ser Gln Ser Glu Ile Gly
Asp Ala Ser
            35                  40                  45

Arg Val Arg Val Ser Gly Gln Gly Leu His Glu Gly His
Thr Phe Glu
        50                  55                  60

Pro Ala Glu Phe Ile Ile Asp Thr Arg Asp Ala Gly Tyr
Gly Gly Leu
65                  70                  75
80

Ser Leu Ser Ile Glu Gly Pro Ser Lys Val Asp Ile Asn
Thr Glu Asp
                85                  90
95

Leu Glu Asp Gly Thr Cys Arg Val Thr Tyr Cys Pro Thr
Glu Pro Gly
                100                 105
110

Asn Tyr Ile Ile Asn Ile Lys Phe Ala Asp Gln His Val
Pro Gly Ser
            115                 120                 125

Pro Phe Ser Val Lys Val Thr Gly Glu Gly Arg Val Lys
Glu Ser Ile
        130                 135                 140

Thr Arg Arg Arg Arg Ala Pro Ser Val Ala Asn Val Gly
Ser His Cys
145                 150                 155
160

Asp Leu Ser Leu Lys Ile Pro Glu Ile Ser Ile Gln Asp
Met Thr Ala
                    165                 170
175

Gln Val Thr Ser Pro Ser Gly Lys Thr His Glu Ala Glu
Ile Val Glu
                180                 185
190

Gly Glu Asn His Thr Tyr Cys Ile Arg Phe Val Pro Ala
Glu Met Gly
            195                 200                 205

Thr His Thr Val Ser Val Lys Tyr Lys Gly Gln His Val
Pro Gly Ser
        210                 215                 220

Pro Phe Gln Phe Thr Val Gly Pro Leu Gly Glu Gly Gly
Ala His Lys
    225                 230                 235
240

Val Arg Ala Gly Gly Pro Gly Leu Glu Arg Ala Glu Ala
Gly Val Pro
                    245                 250
255

Ala Glu Phe Ser Ile Trp Thr Arg Glu Ala Gly Ala Gly
Gly Leu Ala
                260                 265
270

Ile Ala Val Glu Gly Pro Ser Lys Ala Glu Ile Ser Phe
Glu Asp Arg
            275                 280                 285

Lys Asp Gly Ser Cys Gly Val Ala Tyr Val Val Gln Glu
Pro Gly Asp
        290                 295                 300

Tyr Glu Val Ser Val Lys Phe Asn Glu Glu His Ile Pro
Asp Ser Pro
    305                 310                 315
320

Phe Val Val Pro Val Ala Ser Pro Ser Gly Asp Ala Arg
Arg Leu Thr
                    325                 330
335

Val Ser Ser Leu Gln Glu Ser Gly Leu Lys Val Asn Gln
Pro Ala Ser
                340                 345
350

Phe Ala Val Ser Leu Asn Gly Ala Lys Gly Ala Ile Asp
Ala Lys Val

355 360 365
His Ser Pro Ser Gly Ala Leu Glu Glu Cys Tyr Val Thr
Glu Ile Asp
370 375 380
Gln Asp Lys Tyr Ala Val Arg Phe Ile Pro Arg Glu Asn
Gly Val Val
385 390 395
400
Leu Ile Asp Val Lys Phe Asn Gly Thr His Ile Pro Gly
Ser Pro Phe
405 410
415
Lys Ile Arg Val Gly Glu Pro Gly His Gly Gly Asp Pro
Gly Leu Val
420 425
430
Ser Ala Tyr Gly Ala Gly Leu Glu Gly Gly Val Thr Gly
Asn Pro Ala
435 440 445
Glu Phe Val Val Asn Thr Ser Asn Ala Gly Ala Gly Ala
Leu Ser Val
450 455 460
Thr Ile Asp Gly Pro Ser Lys Val Lys Met Asp Cys Gln
Glu Cys Pro
465 470 475
480
Glu Gly Tyr Arg Val Thr Tyr Thr Pro Met Ala Pro Gly
Ser Tyr Leu
485 490
495
Ile Ser Ile Lys Tyr Gly Gly Pro Tyr His Ile Gly Gly
Ser Pro Phe
500 505
510
Lys Ala Lys Val Thr Gly Pro Arg Leu Val Ser Asn His
Ser Leu His
515 520 525
Glu Thr Ser Ser Val Phe Val Asp Ser Leu Thr Lys Ala
Thr Cys Ala
530 535 540
Pro Gln His Gly Ala Pro Gly Pro Gly Pro Ala Asp Ala
Ser Lys Val
545 550 555
560

Val Ala Lys Gly Leu Gly Leu Ser Lys Ala Tyr Val Gly
Gln Lys Ser
565 570
575

Ser Phe Thr Val Asp Cys Ser Lys Ala Gly Asn Asn Met
Leu Leu Val
580 585
590

Gly Val His Gly Pro Arg Thr Pro Cys Glu Glu Ile Leu
Val Lys His
595 600 605

Val Gly Ser Arg Leu Tyr Ser Val Ser Tyr Leu Leu Lys
Asp Lys Gly
610 615 620

Glu Tyr Thr Leu Val Val Lys Trp Gly His Glu His Ile
Pro Gly Ser
625 630 635
640

Pro Tyr Arg Val Val Val Pro
                645

(2) INFORMATION FOR SEQ ID NO: 13:

(i) SEQUENCE CHARACTERISTICS:
(A) LENGTH: 213 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 13:

His Glu Gly Arg Gly Val Thr Gly Asn Pro Ala Glu Phe
Val Val Asn
1 5 10
15

Thr Ser Asn Ala Gly Ala Gly Ala Leu Ser Val Thr Ile
Asp Gly Pro
20 25
30

Ser Lys Val Lys Met Asp Cys Gln Glu Cys Pro Glu Gly
Tyr Arg Val
35 40 45

Thr Tyr Thr Pro Met Ala Pro Gly Ser Tyr Leu Ile Ser
Ile Lys Tyr
50                    55                    60

Gly Gly Pro Tyr His Ile Gly Gly Ser Pro Phe Lys Ala
Lys Val Thr
65                    70                    75
80

Gly Pro Arg Leu Val Ser Asn His Ser Leu His Glu Thr
Ser Ser Val
                    85                    90
95

Phe Val Asp Ser Leu Thr Lys Ala Thr Cys Ala Pro His
His Gly Ala
                    100                    105
110

Pro Gly Pro Gly Pro Ala Asp Ala Ser Lys Val Val Ala
Lys Gly Leu
          115                    120                    125

Gly Leu Ser Lys Ala Tyr Val Cys His Lys Ser Ser Phe
Thr Val Asp
     130                    135                    140

Cys Ser Lys Ala Cys Ile Ile Met Leu Leu Val Gly Val
His Gly Pro
145                    150                    155
160

Trp Thr Pro Cys Asp Glu Ile Leu Val Lys Ala Arg Gly
Gln Pro Ala
                    165                    170
175

Leu Gln Arg Val Leu Thr Cys Phe Lys Asp Lys Gly Glu
Val His Thr
                    180                    185
190

Gly Gly Gln Asn Gly Gly Asp Tyr Gln Ile Pro Cys Lys
Pro Leu Pro
          195                    200                    205

Leu Cys Gly Cys Pro
     210

(2) INFORMATION FOR SEQ ID NO: 14:

     (i) SEQUENCE CHARACTERISTICS:
          (A) LENGTH: 213 amino acids
          (B) TYPE: amino acid
          (C) STRANDEDNESS: unknown
          (D) TOPOLOGY: unknown

    (ii) MOLECULE TYPE: protein

(iii) HYPOTHETICAL: NO

(xi) SEQUENCE DESCRIPTION: SEQ ID NO: 14:

His Glu Gly Arg Pro Thr Glu Pro Gly Asn Tyr Ile Ile
Asn Ile Lys
1               5                   10
15

Phe Ala Asp Gln His Val Pro Gly Ser Pro Phe Ser Val
Lys Val Thr
                20                  25
30

Gly Glu Gly Arg Val Lys Glu Ser Ile Thr Arg Arg Arg
Arg Ala Pro
            35                  40                  45

Ser Val Ala Asn Val Gly Ser His Cys Asp Leu Ser Leu
Lys Ile Pro
        50                  55                  60

Glu Ile Ser Ile Gln Asp Met Thr Ala Gln Val Thr Ser
Pro Ser Gly
65                  70                  75
80

Lys Thr His Glu Ala Glu Ile Val Glu Gly Glu Asn His
Thr Tyr Cys
                85                  90
95

Ile Arg Phe Val Pro Ala Glu Met Gly Thr His Thr Val
Ser Val Lys
                100                 105
110

Tyr Lys Gly Gln His Val Pro Gly Ser Pro Phe Gln Phe
Thr Val Gly
            115                 120                 125

Pro Leu Gly Glu Gly Gly Ala His Xaa Val Arg Ala Gly
Gly Pro Gly
        130                 135                 140

Leu Xaa Lys Ser Ser Trp Ser Ala Ser Arg Ile Gln Tyr
Leu Gly Pro
145                 150                 155
160

Gly Lys Leu Val Leu Glu Ala Trp Pro Leu Leu Ser Xaa
Ala Pro Ala
                165                 170
175

Xaa Leu Xaa Ser Leu Leu Arg Thr Ala Arg Thr Ala Pro
Val Val Leu
180 185
190

Leu Met Leu Val Xaa Glu Pro Ser Asp Xaa Asn Pro Xaa
Gln Val Ser
195 200 205

Thr Lys Glu His Xaa
    210

## Claims

1. A recombinant and/or isolated nucleic acid molecule encoding at least a functional part of a member of the family of Nmi-like proteins or at least a functional part of a member of the family of Hou-like proteins or at least a functional part of a member of the family of IFP35-like proteins for use in the induction of apoptosis in a population of cells related to a pathological condition.

2. A use according to claim 1 wherein said nucleic acid molecule comprises at least a functional and specific part of the sequence of figure 1, 2, 4 or 5 or encoding an amino sequence of figure 6 or a sequence at least 60, preferably 70, preferably 90 % homologous with said functional and specific sequence or comprising a sequence hybridizing to any of the aforegoing sequences under stringent conditions.

3. Use according to claim 1 or 2 wherein said nucleic acid comprises an expression vector.

4. Use according to anyone of the aforegoing claims whereby said cells are provided with apoptosis inducing activity.

5. Use according to claim 4 whereby said apoptosis inducing activity is apoptin-like activity.

6. Use according to any of claims 1-5 wherein said nucleic acid is part of a gene delivery vehicle.

7. A recombinant and/or isolated nucleic acid molecule encoding an Nmi/Hou-like protein comprising at least a functional and/or specific part of the sequence of figure 1 or figure 2 or a sequence at least 60, preferably 70, more preferably 80% homologous therewith.

8. A recombinant and/or isolated nucleic acid molecule encoding an IFP35-like protein comprising at least a functional and/or specific part of the sequence of figure 4 or figure 5 or encoding at least a functional and/or specific part of the amino acid sequence of figure 6 or a sequence at least 60, preferably 70, more preferably 80% homologous therewith.

9. An expression vector comprising a recombinant nucleic acid molecule according to claim 7 and/or 8.

10. An expression vector according to claim 9 further comprising a sequence encoding apoptotic activity.

11. An expression vector according to claim 10 wherein said sequence encoding apoptotic activity encodes apoptin or a functional fragment and/or equivalent thereof.

12. A gene delivery vehicle comprising a recombinant nucleic acid molecule according to claim 7 or 8 or an expression vector according to anyone of claims 9-11.

13. A recombinant or isolated proteinaceous substance comprising at least a functional part of a member of the family of Nmi/Hou-like proteins or at least a functional part of a member of the family of Hou-like proteins for use in the induction of apoptosis in a population of cells related to a pathological condition.

14. An Nmi/Hou-like proteinaceous substance having at least a functional and/or specific part of the sequence of figure 3 or being encoded by a functional and/or specific part of the sequence of figure 1 or figure 2 or being at least 60, preferably 70, preferably 80% homologous to at least a functional and/or specific part of the sequence of figure 3 or being at least 60, preferably 70, preferably 80% homologous to a protein encoded by at least a functional and/or specific part of the sequence of figure 1 or figure 2.

15. A recombinant or isolated proteinaceous substance comprising at least a functional part of a member of the family of Nmi/Hou-like proteins or at least a functional part of a member of the family of Hou-like proteins for use in the induction of apoptosis in a population of cells related to a pathological condition.

16. An IFP35-like proteinaceous substance having at least a functional and/or specific part of the sequence of figure 6 or 7 or being encoded by a functional and/or specific part of the sequence of figure 4 or figure 5 or being at least 60, preferably 70, preferably 80% homologous to at least a functional and/or specific part of the sequence of figure 6 or 7 or being at least 60, preferably 70, preferably 80% homologous to a protein encoded by at least a functional and/or specific part of the sequence of figure 4 or figure 5.

17. A method for inducing apoptosis in cells comprising providing said cells with Nmi/Hou-like protein activity and/or IFP-35-like activity together with apoptin-like activity.

18. Use of apoptin to find proteinaceous substances associated with apoptosis.
